# EUROPEAN PATENT SPECIFICATION

(11) **EP 4 334 423 B1**
(45) Date of publication and mention of the grant of the patent: **02.07.2025**
(21) Application number: 22723796.3
(22) Date of filing: 05.05.2022
(51) Int. Cl.: C11D 1/72, C07C 41/03

(54) **PROCESS FOR ETHOXYLATION OF A MIXTURE OF STRAIGHT CHAIN FATTY ALCOHOL AND BRANCHED CHAIN FATTY ALCOHOL IN A SINGLE STAGE REACTION**
VERFAHREN ZUR ETHOXYLIERUNG EINES GEMISCHS AUS GERADKETTIGEM FETTALKOHOL UND VERZWEIGTKETTIGEM FETTALKOHOL IN EINER EINSTUFIGEN REAKTION
PROCÉDÉ D'ÉTHOXYLATION D'UN MÉLANGE D'ALCOOL GRAS À CHAÎNE DROITE ET ALCOOL GRAS À CHAÎNE RAMIFIÉE DANS UNE RÉACTION EN UNE SEULE ÉTAPE

(30) Priority: 05.05.2021 EP 21172275
(43) Date of publication of application: 13.03.2024
(73) Proprietor: SABIC Global Technologies B.V., 4612 PX Bergen op Zoom (NL)
(72) Inventor: AL-ANAZI, Flaiyh Farhan N., Riyadh Riyadh, 11551 (SA); CHIDAMBARA IYER, Narayan Karumanassery, Riyadh Riyadh, 11551 (SA); GHAZWANI, Qasem Ahmed A., Riyadh Riyadh, 11551 (SA)
(74) Representative: Hoeger, Stellrecht & Partner Patentanwälte mbB
(86) International application number: PCT/IB2022/054183
(87) International publication number: WO 2022/234523

(56) References cited:
- EP-A1- 0 572 453
- WO-A1-2007/096292
- WO-A1-2018/078603
- DE-A1- 4 004 379
- GB-A- 1 280 563
- US-A1- 2008 188 396

## Description

### TECHNICAL FIELD

The disclosure concerns methods of ethoxylating combinations of alcohols to provide desired effectiveness as a detergent.

### BACKGROUND

Alcohol alkoxylates are conventionally prepared via the condensation reaction of an alcohol and an alkylene oxide in the presence of a suitable catalyst. The obtained alcohol alkoxylate is more precisely described as a mixture comprising a number of alcohol derivatives with a varying alkoxylate content, or more specifically as a mixture of alcohol derivatives having a variety of alkylene oxide or ethylene oxide units present. Typically, the number of ethylene oxide units present per molecule defines the average number of ethylene oxide units present per molecule, which may be referred to as an average number of ethoxylation. Such alcohol alkoxylates have been widely known as surfactants. Often, the average EO content of the alcohol ethoxylate may vary the properties of the given surfactant. Industrial methods to prepare these alcohol ethoxylates, especially for mass production as surfactants, may require a number of discrete processes to form the desired alkoxylated alcohols.

There remains a need in the art for streamlined methods of formulating alcohol ethoxylates. These and other needs are addressed in the present disclosure.

### SUMMARY

The present disclosure relates to methods of forming ethoxylated alcohol composition as defined in the claims. The method may comprise combining, in a single reaction vessel, a C₁₂₋₁₄ alcohol, isodecyl alcohol, and a catalyst to provide an isodecyl- C₁₂₋₁₄ alcohol mixture. The C₁₂₋₁₄ alcohol and isodecyl alcohol are combined in a weight ratio of from about 0.81:1 to 1.4:1 of C₁₂₋₁₄ alcohol to isodecyl alcohol. The isodecyl- C₁₂₋₁₄ alcohol mixture may be dehydrated, and ethylene oxide may be added in an amount of about 2 moles to about 12 moles of ethylene oxide per total mole of alcohol to provide an ethoxylated alcohol composition. The ethoxylated alcohol composition effects a detergency measured as a reflectance at 460 nm of a stained fabric at which the ethoxylated alcohol composition has been applied that is within 5% of a reflectance observed at 460 nm at the stained fabric at which a reference composition has been applied, wherein the reference composition comprises a 1:1 by weight mixture of isodecyl alcohol ethoxylate and lauryl alcohol ethoxylate.

While aspects of the present disclosure may be described and claimed in a particular statutory class, such as the system statutory class, this is for convenience only and one of skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

Additional aspects of the disclosure will be set forth in part in the description which follows, and in part will be obvious from the description, or can be learned by practice of the disclosure. The advantages of the disclosure will be realized and attained by means of the elements and combinations particularly pointed out in the appended claims. It is to be understood that both the foregoing general description and the following detailed description are exemplary and explanatory only and are not restrictive of the disclosure, as claimed.

### BRIEF DESCRIPTION OF THE FIGURES

The accompanying figures, which are incorporated in and constitute a part of this specification, illustrate several aspects and together with the description serve to explain the principles of the disclosure.
FIG. 1 is a graphical representation of values for cloud point, wetting, and surface tension for ethoxylated alcohol mixtures of varying ratios.
FIG. 2 is a graphical representation of the cloud point, wetting, and surface tension for ethoxylated alcohol mixtures of varying ratios.
FIG. 3 is a graphical representation of the reflectance observed at 460 nm for ethoxylated alcohol mixtures of varying ratios at 40 °C.
FIG. 4 is a graphical representation of the reflectance observed at 460 nm for ethoxylated alcohol mixtures of varying ratios at 50 °C.

### DETAILED DESCRIPTION

Industrial methods to prepare alcohol ethoxylate compositions, especially for mass production as surfactants, may require a number of discrete processes to form the desired alkoxylated alcohols including distillation, separation, etc.

Conventional methods of preparing ethoxylated alcohols are widely known. However, ethoxylation of a mixture of straight chain and branched primary alcohols in a single reactor vessel or single stage reaction remains elusive. Traditional methods involve multiple steps, or sub-steps, for combining and then purifying and/or distilling a mixture of ethoxylated alcohols.

U.S. Patent No. 3,682,849 discloses the preparation of ethoxylated alcohol mixtures that are useful as surfactants. The alcohol ethoxylate compositions are prepared in a multistage process including distillation.

United Kingdom Patent Publication No. 1542696 discloses liquid detergent compositions produced by the condensation of alcohols. The primary and secondary alcohols disclosed are condensed; the ethoxylated alcohols formed are subsequently mixed to provide the liquid detergent compositions.

US Patent No. 7,906,474 discloses surfactants derived from a mixture of straight chain alcohols that have been propoxylated and ethoxylated.

US Patent No. 9,334,213 discloses alkoxylation of perfluoropolyether alcohols rather than hydrocarbon-based alcohols.

US 2008/188396 A1 discloses a method of ethoxylating an alcohol mixture.

Alkoxylated alcohols represent an industrially important class of nonionic surfactants. Detergents often include mixed ethoxylates as non-ionic surfactants in addition to other common detergent components such as, for example, anionic surfactants, cationic surfactants, enzymes, polycarbonates, optical brighteners. In some aspects, the disclosed methods provide ethoxylated blends of alcohols exhibiting comparable detergency and anti-re-deposition properties to physical mixtures of analogous ethoxylated alcohols. The means of obtaining a detergent composition may thus be simplified but may maintain efficiency as a detergent formulation when compared to a physical mixture of ethoxylated alcohols. The methods disclosed herein provide detergent compositions comprising an ethoxylated combination of alcohols obtained according to a single stage process. Precursor alcohols may be combined in a single reactor vessel and subsequently ethoxylated in the presence of a suitable catalyst under conditions effective to provide the ethoxylated alcohol compositions of the present disclosure. These conditions are characterized according to a particular ratio of the precursor alcohols, temperature, pressure, and an *in-situ* hydroxyl value, among other factors.

According to various aspects of the present invention, a C₁₂₋₁₄ alcohol and isodecyl alcohol are combined in a single reaction vessel in a ratio of from about 0.8:1 to 1.4:1 of C₁₂₋₁₄ alcohol to isodecyl alcohol with a suitable catalyst to provide an isodecyl- C₁₂₋₁₄ alcohol mixture. The isodecyl- C₁₂₋₁₄ alcohol mixture may be subjected to dehydration prior to the addition of ethylene oxide in an amount of about 2 moles to about 12 moles of ethylene oxide per mole of alcohol to provide the disclosed ethoxylated alcohol composition,

As provided herein, the present disclosure provides a straightforward, single stage ethoxylation of a mixture of alcohols, or mixture of precursor alcohols as referenced herein. Precursor alcohols may thus be combined in a single reaction vessel with a suitable catalyst prior to ethoxylation. The precursor alcohol of the present disclosure may refer to primary alcohols of the general formula ROH, wherein R is a C₁₋₂₀ alkyl group, a C₃₋₁₈ cycloalkyl group, a (linear or branched C₁₋₁₂ alkyl) alkyl group.

The alcohol mixture may comprise at least a first precursor alcohol and a second precursor alcohol. The precursor alcohols may feature a combination of straight (or linear) and branched primary alcohols. A linear or straight chain primary alcohol may correspond to formula 1.

R-[CH₂]ₘ-OH (1)

wherein R is a linear hydrocarbyl or alkyl group, wherein m is an integer between, for example 8 and 20, and wherein each carbon atom is bound to its two neighbors and to two hydrogen atoms and wherein the terminal carbon atom is bound to one carbon atom and three hydrogen atoms. The linear hydrocarbyl group may contain between 7 and 16 carbon atoms. A linear alcohol may include lauryl alcohol ethoxylate. As an example, a first precursor alcohol may comprise about 75% C₁₂ straight chain alcohol and 25% C₁₄ straight chain alcohol, referred to herein as a C₁₂₋₁₄ aliphatic straight chain alcohol. A branched chain primary alcohol may be represented as in formula 1 wherein R includes a branched hydrocarbyl moiety. For the branched alcohol, the branched hydrocarbyl moiety (R) may contain between 5 and 16 carbon atoms. A second precursor alcohol may comprise a C₁₀ aliphatic branched chain alcohol such as isodecyl alcohol.

The precursor alcohols are combined in the single reaction vessel in a particular ratio According to the invention, the first precursor alcohol and second precursor alcohol are combined in a particular weight ratio of from about 0.8:1 to 1.4:1.

The C₁₂₋₁₄ alcohol and C₁₀ alcohol may be combined in a ratio of from about 0.8:1 to 1.2:1. In a further aspect, the C₁₂₋₁₄ alcohol and C₁₀ alcohol may be combined in a 1:1 ratio. As a specific example, a weight ratio of alcohols may be 45 C₁₂₋₁₄ alcohol: 55 C₁₀ alcohol. As a further example, the mole ratio of precursor alcohols may be 40 C₁₂₋₁₄ alcohol: 60 C₁₀ alcohol.

Precursor alcohols are combined in a single reaction vessel with a suitable catalyst. Suitable catalysts may include alkali metal hydroxides, including, but not limited to, potassium hydroxide, sodium hydroxide, sodium methoxide or a combination thereof. The catalyst may be combined with the precursor alcohols in an amount of from about 0.05 % to about 0.6 %. For example, the catalyst may be used in an amount of 0.2 %.

The mixture of precursor alcohols and catalyst may be dehydrated. As an example, the mixture may be dried, as by vapor phase removal of any water present. Conventionally, methods for vapor-phase removal of the light components of the initial ethoxylation product mixture may be employed. These methods for vapor-phase removal of the light components conventionally include distillation and evaporation conducted at pressures which are at or below atmospheric. According to the present methods, dehydration may proceed in the same reaction vessel and may comprise purging the vessel with nitrogen, heating to 120 °C, and applying a vacuum. This may allow for a single stage process.

Conventional methods may require a distillation after the reaction as a purification process. The methods of the present disclosure however do not require distillation or a purification process. As such, methods of the present disclosure proceed in a single stage or single reactor vessel.

Ethoxylating serves to introduce a desired average number of ethylene oxide units per alcohol ethoxylate molecule. As provided herein, ethoxylating may comprise combining the alcohol mixture with a catalyst and reacting the resulting mixture with about 6 moles of ethylene oxide per 1 mole of alcohol. That is, ethylene oxide in a prescribed amount may be added to the dehydrated mixture to commence the ethoxylating reaction. The amount of ethylene oxide added may be calculated so as to provide from about 2 to about 12 moles of ethylene oxide per mole of alcohol. Ethylene oxide is thus introduced in an amount of about 2 moles to about 12 moles, or from about 2 moles to about 8 moles of ethylene oxide per mole of alcohol in the dehydrated mixture. As a specific example, an average of about 6, about 6.2, or about 6.3 moles of ethylene oxide per mole of alcohol is introduced to the mixture for the ethoxylating reaction.

The resulting mixture is allowed to react until the ethylene oxide is consumed, the course of the reaction being accompanied by a decrease in reaction pressure. More specifically, the ethoxylation reaction may proceed until a particular hydroxyl value in the mixture is obtained. Hydroxyl value may refer to a measure of the content of free hydroxyl groups in a chemical substance, usually expressed in units of the mass of potassium hydroxide (KOH) in milligrams equivalent to the hydroxyl content of one gram of the chemical substance. The value is important because it helps determine the Stoichiometry of a reaction (ethoxylation reaction). The value also be used to calculate the molecular weight. Hydroxyl value is linked with the ethylene oxide and hydrocarbon ratio. The hydroxyl value may also be used to control the molecular weight and achieve the desired hydrophilic-lipophilic balance (HLB) value. HLB number may be defined as the ratio of hydrophile group to lipophile group and generally indicates whether a surfactant will promote water-in-oil or oil-in-water emulsion. An HLB values less than 6 may be considered lipophilic and favors stabilization of water-in-oil emulsification, while values from 10 to 18 may be hydrophilic and can favor oil-in-water emulsification.

The ethoxylation reaction may proceed until a desired hydroxyl value is obtained. As a specific example, ethoxylating proceeds until a hydroxyl value of from 122-132 is observed when determined in accordance with ASTM E1899-08.

The ethoxylation reaction may be conducted at an elevated temperature and pressure. Suitable reaction temperatures are from about 120 °C to about 220 °C, or from about 140 °C to about 160 °C, or from about 150 °C to about 170 °C. A suitable reaction pressure may be achieved by introducing to the reaction vessel the required amount of ethylene oxide which has a high vapor pressure at the desired reaction temperature. The ethoxylation reaction may proceed at a particular pressure in the single reactor vessel. More specifically, the ethoxylating may proceed at a pressure of about 2 bar to about 3.5 bar. The pressure may serve as a measure of the degree of reaction and the reaction is considered to be substantially complete when the pressure no longer decreases with time.

An ethoxylated mixture of alcohols is obtained following digestion, a process control check, and cooling and neutralization. Digestion may be performed in the same reaction vessel at 160-180 °C. A process control check may be performed to ensure completion of reaction by checking the available hydroxyl in the reaction vessel. Once the desired hydroxyl number is achieved, the obtained product is cooled and then neutralized (for example, using acetic acid) to a desired pH (4-10).

The obtained ethoxylated mixture of alcohols achieved according to the methods disclosed herein may be efficient as a surfactant or a detergent. The mixture does yield a detergency measured as a reflectance at 460 nm of a laundered stained fabric, which has been treated with the ethoxylated alcohol composition, that is within 5% of a reflectance observed at 460 nm at the stained fabric which has been treated with a reference composition, wherein the reference composition comprises a 1:1 physical mixture of isodecyl alcohol ethoxylate and lauryl alcohol ethoxylate. The stained fabric may be laundered, or treated, with the composition. A physical mixture of isodecyl alcohol ethoxylate and lauryl alcohol ethoxylate may refer to a combination of separately alkoxylated alcohols that have been physically combined as opposed to the present composition comprising a mixture of precursor alcohols that have been subjected to an ethoxylating reaction in a single stage process.

As used herein, an alkoxylated alcohol or alcohol alkoxylate may refer to and may be characterized by the structure as in formula 2. 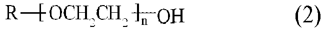 wherein R comprises a hydrocarbyl or alkyl moiety and wherein n corresponds to the number of moles of ethylene oxide (or ethyleneoxy, EO) present per mole of alcohol. The EO units correspond to the hydrophilic or water-soluble portions of the surfactant molecule. Wherever a degree of alkoxylation (or ethoxylation) is discussed the numbers referred to are molar average numbers, essentially corresponding to the reaction of the indicated number of moles of alkylene (or ethylene) oxide with one mole of alcohol.

Branched chain ethoxylated alcohols may be represented as in formula 1 where R includes a branched hydrocarbyl moiety. For the branched ethoxylated alcohol, the branched hydrocarbyl moiety (R) may contain between 5 and 16 carbon atoms. As an example, a branched ethoxylated alcohol may be characterized by formula 3.

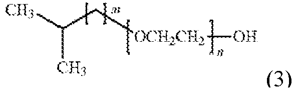

A linear or straight chain ethoxylated alcohol may correspond to formula 1 where R is a linear hydrocarbyl or alkyl group wherein each carbon atom is bound to its two neighbors and to two hydrogen atoms and wherein the terminal carbon atom is bound to one carbon atom and three hydrogen atoms. The linear hydrocarbyl group may contain between 7 and 16 carbon atoms. An example of a linear ethoxylated alcohol may include lauryl alcohol ethoxylate. A linear ethoxylated alcohol may include a compound having the structure according to formula 4. 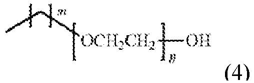 wherein m is an integer between, for example, 8 and 14 and n corresponds to units of ethylene oxide EO.

### Detergent properties of composition obtained by disclosed method

Detergency and re-deposition may be assessed based upon the reflectance observed at a stained fabric and the reflectance observed at the stained fabric after an application of a given detergent composition. Performance as a detergent (detergency) may be evaluated according to the principle that a deposit of material (or a stain) on a fabric would result in a lower reflectance of light observed at the fabric. In turn, the removal of such a deposit would increase the reflectance of light observed at the fabric. Thus, a high reflection of light means the stain has been removed to a greater extent indicating a better detergent performance of the composition. Detergency of the compositions may be determined using aqueous solutions comprising the compositions described herein. A stained fabric treated with an application of the disclosed composition may exhibit higher reflectance values than a fabric treated with an individual alcohol ethoxylate or other combinations of alcohol ethoxylates. With respect to anti-redeposition properties, a white fabric included with the stained fabrics in an application of the disclosed solution may also exhibit higher reflection values.

The stained fabric may be laundered, or treated, with the reference composition, which is a physical mixture of isodecyl alcohol ethoxylate and lauryl alcohol ethoxylate. This combination may be in the presence of a builder for a laundry wash trial to assess detergency. An example of a laundry wash trial may include the laundering of soiled fabrics in deionized water at 40 °C with a dosage of 0.4 % of the disclosed detergent composition in a 25 grams per liter load. The wash trial may proceed in a tergotometer for example. The tergotometer may operate at 200 revolutions per minute (rpm) for a duration of 30 minutes. Upon completion of laundering, the fabric may be dried at room temperature.

In a further example, the ethoxylated alcohol compositions formed according to the methods described herein also exhibited comparable detergency to common nonionic surfactant nonyl phenol ethoxylate (NP9). Surfactant formulations formed according to the methods of the present disclosure may be particularly useful as NP9 and NP9-based surfactants face increasing environment scrutiny. The disclosed surfactant compositions may exhibit a detergency and storage stability comparable to conventional surfactant NP9.

The methods of the present disclosure does yield surfactant or detergent compositions that provide a detergency measured according to the relevant performance of the surfactant composition when applied to soiled fabric in a laundry wash trial. A stained fabric subjected to a laundry wash trial with the surfactant composition does exhibit a reflectance at 460 nm that is within 5% of a reflectance observed at 460 nm on a stained fabric subjected to a laundry wash trial with a reference composition, wherein the reference composition comprises a 1:1 physical mixture of ethoxylated C₁₀ aliphatic branched chain alcohol having 5 moles ethoxylate and ethoxylated C₁₂₋₁₄ aliphatic straight chain alcohol having 7 moles ethoxylate. The laundry wash trial may comprise laundering of the soiled fabric at 40 °C with a dosage of 0.2 % of the surfactant or reference composition in accordance with ASTM-3050-70. In a further example, a stained fabric subjected to a laundry wash trial with the surfactant composition may exhibit a reflectance at 460 nm that is within 1% of the reflectance observed at 460 nm on a stained fabric subjected to a laundry wash trial with a reference composition, wherein the reference composition comprises an ethoxylated mixture of C₁₀ aliphatic branched chain alcohol having 5 moles ethoxylate and ethoxylated C₁₂₋₁₄ aliphatic straight chain alcohol having 7 moles ethoxylates, or wherein the reference composition comprises an ethoxylated mixture of C₁₀ aliphatic branched chain alcohol having 3 moles ethoxylate and C₁₂₋₁₄ aliphatic straight chain alcohol having 7 moles ethoxylates, wherein the laundry wash trial comprises laundering of the fabric in deionized water at 40 °C with a dosage of 0.4 % of the surfactant or reference composition, and wherein the stained fabric comprises pigment lanolin wfk 20C, pigment sebum wfk 20D, egg yolk 20EG, or lipstick wfk 20 LS.

As a yet further example, the surfactant composition may exceed the performance of a reference composition comprising a physical mixture of ethoxylated alcohols in a laundry wash trial with respect to certain stain types. Specifically, a stained fabric subjected to a laundry wash trial with the surfactant composition may exhibit a reflectance at 460 nm that is greater than the reflectance observed at 460 nm on a stained fabric subjected to a laundry wash trial with a reference composition, wherein the reference composition comprises an ethoxylated mixture of C₁₀ aliphatic branched chain alcohol having 5 moles ethoxylate and ethoxylated C₁₂₋₁₄ aliphatic straight chain alcohol having 7 moles ethoxylates, or wherein the reference composition comprises an ethoxylated mixture of C₁₀ aliphatic branched chain alcohol having 3 moles ethoxylate and C₁₂₋₁₄ aliphatic straight chain alcohol having 7 moles ethoxylates, wherein the laundry wash trial comprises laundering of the fabric in deionized water at 50 °C with a dosage of 0.4 % of the surfactant or reference composition, and wherein the stained fabric comprises pigment lanolin wfk 20C, pigment sebum wfk 20D, egg yolk 20EG, or lipstick wfk 20 LS.

Regarding storage stability, the methods of the present disclosure provide surfactant compositions that may exhibit comparable storage stability to NP9 surfactant because the surfactant compositions formed have a cloud point similar to that of NP9. Cloud point may refer to the temperature above which an aqueous solution of a water-soluble surfactant becomes turbid. As such, considering the cloud point is generally essential to assessing storage stability because storing formulations at temperatures significantly higher than the cloud point may result in phase separation and surfactant instability. Generally, nonionic surfactants show optimal effectiveness when used near or below their cloud point. Surfactant compositions formed according to the methods disclosed herein may have a cloud point within 5 %, within 3%, within 2 %, or within 1% of the cloud point of NP9 at the same given temperature.

The detergent efficacy of the compositions prepared according to the methods described herein may be evaluated against a number of stain types. In some aspects, the soiled or stained fabric may be a fabric having a stain type according to any one of a number of WFK Testgewebe GmbH stain types or an EMPA of Switzerland standard stain type. For example, the stain may comprise one or more of one of the following stain types soy sauce pigment; olive oil WFK 20B, pigment lanolin WFK 20C, pigment sebum WFK 20D, egg yolk WFK 20EG, used motor oil WFK 20GM, Coca Cola^{™} WFK 20H, coffee WFK 20K, red grape WFK 20LIU, lip stick WFK 20LS, makeup WFK 20MU, pigment egg WFK 20N, pigment vegetable fat WFK 20PF, soot mineral oil WFK 20RM, tomato bf sauce WFK 20SG, spinach WFK 20SP, ketchup WFK 20T, curry WFK 20U, soy sauce WFK 20V, chocolate WFK 20Z, blood-milk-C EMPA 117, for stained or soiled fabric according to WFK Testgewebe GmbH or blood-milk C EMPA 117 according to EMPA of Switzerland standard stain type. In specific examples, the stain may comprise one or more of the following stain types pigment lanolin WFK 20C, pigment sebum WFK 20D, egg yolk WFK 20EG, and lip stick WFK 20LS.

The white fabric may be a white fabric according to WFK Testgewebe GmbH WFK 20A. As an example, the white fabric used may be a cotton-polyester blend, such as a 70% cotton, 30 % polyester blend.

Surfactant compositions of the present disclosure may also perform comparably or better than surfactant compositions comprising merely a physical mixture of individually ethoxylated alcohols. In an aspect, a stained fabric subjected to a laundry wash trial with the surfactant composition may exhibit a reflectance at 460 nm that is within 1% of a reflectance observed at 460 nm on a stained fabric subjected to a laundry wash trial with a reference composition. The reference composition does comprise an ethoxylated mixture of C10 aliphatic branched chain alcohol having 5 moles ethoxylate and ethoxylated C12-14 aliphatic straight chain alcohol having 7 moles ethoxylates.

To assess anti-redeposition performance, a white fabric may be added along with the stained fabrics during a treatment of the stained fabric with a given detergent. Treatment may comprise a laundry wash trial comprising the detergent. Reflectance of the unwashed, non-stained white fabric may be observed prior to a laundering and then observed after the wash trial with a given stained fabric. Reflectance values closer to that observed for the unwashed fabric may indicate that the detergent formulation is a suitable anti-redeposition agent. In some aspects, a white fabric included with the stained fabrics during a treatment of the disclosed solution may also exhibit higher reflection values than a white fabric included with the stained fabrics during a treatment with an alternate solution.

The compositions disclosed herein may be provided in liquid form or can be provided in solid or powdered form. In some examples, the provided cleansing compositions are formulated as a heavy-duty detergent powder, heavy-duty detergent liquid, dishwashing liquid, machine dishwashing detergents, institutional detergents, detergent liquids, laundry aid, pretreatment aid, after-treatment aids, presoaking product, hard surface cleaner, or carpet cleaner.

For laundry wash trials, a builder may be included with disclosed composition comprising the ethoxylated mixture of alcohols. Builders have a number of functions including softening, buffering, emulsifying, and removal of multivalent cations from water. Builders may accomplish these functions by, for example, sequestration, holding metal ions in solution; or by precipitation, removing ions from solution as insoluble material (precipitate). The builder may be an organic compound or an inorganic compound.

Organic builders may be used in minor amounts as supplements to inorganic builders such as phosphates and zeolites. Builders whether inorganic or organic, may be present as an alkali metal salt, such as sodium salt. Suitable builders may be used in amounts of from 1 wt. % to 30 wt. %, more specifically from 10 wt. % to 25 wt. %. An example of a suitable builder is sodium tripolyphosphate, which may be used in combination with sodium orthophosphate, and/or sodium pyrophosphate.

The pH of the compositions provided herein, as measured by a conventional pH meter at room temperature (RT) is from about 4 to about 10, more specifically from 4 to 9, or from 4 to 7.

A laundry wash trial as described herein may be performed at a desired temperature. In specific examples, the laundry wash trial may be performed at greater than 30 °C, for example at 40 °C (or about 40 °C) or at 50 °C (or about 50 °C).

### DEFINITIONS

It is to be understood that the terminology used herein is for the purpose of describing particular aspects only and is not intended to be limiting. As used in the specification and in the claims, the term "comprising" may include the aspects "consisting of" and "consisting essentially of." Unless defined otherwise, all technical and scientific terms used herein have the same meaning as commonly understood by one of ordinary skill in the art to which this disclosure belongs. In this specification and in the claims, which follow, reference will be made to a number of terms which shall be defined herein. All publications mentioned herein are incorporated herein by reference to disclose and describe the methods and/or materials in connection with which the publications are cited.

As used in the specification and the appended claims, the singular forms "a," "an" and "the" include plural referents unless the context clearly dictates otherwise. Thus, for example, reference to "a polycarbonate" includes mixtures of two or more such polycarbonates. Furthermore, for example, reference to a filler includes mixtures of two or more such fillers.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "optional" or "optionally" mean that the subsequently described event, condition, component, or circumstance may or may not occur, and that the description includes instances where said event or circumstance occurs and instances where it does not.

The term "anti-redeposition" as used herein may refer to the capability of a composition or detergent formulation to facilitate sustained suspension of soils throughout the detergent solution and preventing removed soils/stains from being redeposited onto the substrate being cleaned. Anti-redeposition properties may be evaluated according to a number of standards known in the art including, for example, ASTM D4008 - 95.

"Detergency" may refer to the removal of liquid or solid substances (such as, stains) from a solid surface brought in contact with the sample. In aspects of the present disclosure, detergency may be assessed or evaluated according to the relevant performance of the surfactant composition when applied to soiled fabric in a laundry wash trial. As an example, this performance may be evaluated directly with the reflectance observed at the soiled fabric before and/or after the wash trial.

The term "reflectance" as used herein refers to a ratio of the intensity of light or other radiation to that of the light or other radiation incident on a surface. Reflectance may be presented as a percentage and may be observed according to a number of standards known in the art including, for example, ASTM D 3050-07.

As used herein, the term or phrase "effective," "effective amount," or "conditions effective to" refers to such amount or condition that is capable of performing the function or property for which an effective amount is expressed. As will be pointed out below, the exact amount or particular condition required will vary from one aspect to another, depending on recognized variables such as the materials employed, and the processing conditions observed. Thus, it is not always possible to specify an exact "effective amount" or "condition effective to." However, it should be understood that an appropriate effective amount will be readily determined by one of ordinary skill in the art using only routine experimentation.

As used herein, the term or phrase "effects" or "to effect" indicates that a given component gives rise to or causes a particular outcome or response.

Disclosed are component materials to be used to prepare disclosed compositions of the disclosure as well as the compositions themselves to be used within methods disclosed herein. Thus, if there are a variety of additional steps that can be performed it is understood that each of these additional steps can be performed with any specific aspect or combination of aspects of the methods of the disclosure.

References in the specification and concluding claims to parts by weight, of a particular element or component in a composition or article denotes the weight relationship between the element or component and any other elements or components in the composition or article for which a part by weight is expressed. Thus, in a composition containing 2 parts by weight of component X and 5 parts by weight component Y, X and Y are present at a weight ratio of 2:5 and are present in such ratio regardless of whether additional components are contained in the compound.

A weight percent of a component, unless specifically stated to the contrary, is based on the total weight of the formulation or composition in which the component is included. For example, if a particular element or component in a composition or article is said to have 8% weight, it is understood that this percentage is relation to a total compositional percentage of 100%.

Compounds disclosed herein are described using standard nomenclature. For example, any position not substituted by any indicated group is understood to have its valence filled by a bond as indicated, or a hydrogen atom. A dash ("-") that is not between two letters or symbols is used to indicate a point of attachment for a substituent. For example, -CHO is attached through carbon of the carbonyl group. Unless defined otherwise, technical and scientific terms used herein have the same meaning as is commonly understood by one of skill in the art to which this disclosure belongs.

The term "comparable" as used herein may refer to similarity between given resin compositions described herein. Comparable may be used to express that properties, or the quantified values of given properties, are similar to or commensurate with the properties of another. In some examples, "comparable" may refer to an absolute value proximity to a numerical value according to a percentage.

Without further elaboration, it is believed that one skilled in the art can, using the description herein, utilize the present disclosure. The following examples are included to provide addition guidance to those skilled in the art of practicing the claimed disclosure. The examples provided are merely representative of the work and contribute to the teaching of the present disclosure. Accordingly, these examples are not intended to limit the disclosure in any manner.

While aspects of the present disclosure can be described and claimed in a particular statutory class, such as the system statutory class, this is for convenience only and one of skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

Throughout this application, various publications are Nothing herein is to be construed as an admission that the present disclosure is not entitled to antedate such publication by virtue of prior disclosure. Further, the dates of publication provided herein can be different from the actual publication dates, which can require independent confirmation.

### EXAMPLES

The following examples are put forth so as to provide those of ordinary skill in the art with a complete disclosure and description of how the methods, devices, and systems disclosed and claimed herein are made and evaluated and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in degrees Celsius (°C) or is at ambient temperature, and pressure is at or near atmospheric.

As a comparative composition, isodecyl alcohol 5 moles ethoxylate (DA5) (from synthesis in a pilot plant of 30L capacity) was combined with lauryl alcohol 7 moles ethoxylate (L7) (from synthesis in the pilot plant of 30L capacity) were combined in a 1:1 ratio to provide a physical mixture of ethoxylated alcohols isodecyl alcohol ethoxylate and lauryl alcohol ethoxylate designated as LDBB.

As an inventive example, isodecyl alcohol (IDA) and lauryl alcohol (C₁₂₋₁₄ alcohol) were combined at a weight ratio of 45 C₁₂₋₁₄ alcohol: 55 IDA, corresponding to a molar ratio of 40 C₁₂₋₁₄ alcohol: 60 IDA, in a single reaction vessel. The mixture was then ethoxylated in the pilot plant of 30L capacity to provide LDB1 prepared according to methods of the present disclosure. Table 1 below provides the ethoxylation conditions. The reaction sequence comprised the combination of alcohol precursors IDS and C₁₂₋₁₄ alcohol with a catalyst (potassium hydroxide as a 50% aqueous solution, in an amount of 0.2%), dehydration, addition of ethylene oxide, digestion, a process control check, and cooling and neutralization.

**Table 1. Ethoxylation Conditions to Form LDB1**

| | |
|---|---|
| **Average No. of Moles ethylene oxide per mole of alcohol** | **6.3** |
| Reaction temperature | 150 °C - 170 °C |
| Reaction Pressure | 2.0 bar - 3.0 bar |

Ethoxylation proceeded until a desired hydroxyl value from 122 to 132 was obtained when measured in accordance with ASTM E1899-08 to provide LDB1.

Physical and chemical properties of LDBB and LDB1 were compared. These properties were further compared with Nonyl phenol ethoxylate 9 moles EO (NP9), a conventional ethoxylate detergent and are presented in Table 2. The percent difference (% Diff.) as an absolute value compares LDBB and LDB1.

**Table 2. Physical and Chemical Properties of LDB1, LDBB, and NP9**

| **Properties** | **Standard** | **LDBB (Comparative)** | **LDB1 (Inventive Composition)** | **\|%diff.\| (LDBB to LDB1)** | **NP9 (Standard Conventional Detergent)** |
|---|---|---|---|---|---|
| Appearance | (visual inspection) | Colorless liquid | Colorless liquid | NA | Colorless liquid |
| pH 1% aq. Solution | ASTM D1293-12 | 6.1 | 6.2 | 2% | 6 |
| Surface tension (dynes/cm) | ASTM - D1331-14 | 27.3 | 27.1 | 1% | 32 |
| CMC (ppm) | IS0 4311-1979 | 67 | 67 | 0% | 60 |
| Wettability (0.2%) (sec) | ASTM D2281-10 | 3.7 | 3.61 | 2% | 5.56 |
| Cloud point (1% aq.) (°C) | ASTM D 2024-09 | 46.2 | 48.3 | 5% | 50 |

Detergency properties of LDBB, LDB1 and NP9 were measured using a Tergotometer and Konica Minolta spectrophotometer and are presented in Table 3. Laundry wash trials were conducted using a tergotometer and deionized water at 40 °C. The wash trials were performed at 200 revolutions per minute (rpm) for 30 minutes with a load of 25 grams per liter (g/L) for the varying ethoxylate types. The unwashed U/W fabric is presented as a comparative sample. The laundered samples were rinsed and dried at room temperature. Reflectance was obtained at 460 nm for each sample of laundered fabric according to the stain types. The reflectance measurements were performed on a Konica Minolta Spectrophotometer 3610A. All measurements are made after calibrating the instrument against a black standard and white standard of known reflectance. Measurements were made according to the reference standard ASTM D 3050 - 07. Reflectance values of all the fabrics were obtained before and after washing. Results are presented in Table 3. The percent difference (% Diff.) as an absolute value compares LDBB and LDB1.

**Table 3. Detergency Trial (Stain Removal)**

| **Laundry wash trial 16-25** | | | | | |
|---|---|---|---|---|---|
| **Method : Tergotometer, DIW, 40 °C, 200 rpm, 30 min, load 25g/L, dosage 0.2%, RT drying** | | | | | |
| **Stain type** | **Reflectance (%) at 460 nm** | | | | |
| | **U/W** | **Ethoxylate type** | | | |
| | | **LDB Blend (Comparative)** | **LDB1 (Inventive)** | **\|% Diff.\|** | **NP9 (Standard Conventional Detergent)** |
| **Pigment Olive oil wfk 20B** | 34.9 | 62.75 | 63.01 | 0.4% | 60.13 |
| **Pigment Lanolin wfk 20C** | 34 | 52.52 | 52.95 | 0.8% | 47.31 |
| **Pigment Sebum wfk 20D** | 34.4 | 68.53 | 68.3 | 0.3% | 68.3 |
| **Egg Yolk wfk 20EG** | 47.9 | 60.27 | 61.51 | 2.1% | 56.64 |
| **Used motor oil wfk 20GM** | 30.9 | 52.88 | 52.1 | 1.5% | 47.89 |
| **Coca cola wfk 20H** | 32.9 | 73.61 | 74.04 | 0.6% | 73.53 |
| **Coffee WFK 20K** | 39.2 | 72.95 | 73.43 | 0.7% | 72.87 |
| **Red grape wfk 20LIU** | 43.1 | 69.06 | 70.02 | 1.4% | 69.17 |
| **Lip stick wfk 20LS** | 20.7 | 61.4 | 60.06 | 2.2% | 50.18 |
| **Make up wfk 20MU** | 49.6 | 67.18 | 67.69 | 0.8% | 66.96 |
| **Pigment Egg wfk 20N** | 28.4 | 55.68 | 56.74 | 1.9% | 52.63 |
| **Pigment Veg fat wfk 20PF** | 31.3 | 68.85 | 67.72 | 1.6% | 68.08 |
| **Soot Min Oil wfk 20RM** | 19.6 | 39.55 | 39.89 | 0.9% | 36.28 |
| **Tomato bf sauce wfk 20SG** | 25.3 | 54.15 | 56.68 | 4.7% | 52.84 |
| **Spinach wfk 20SP** | 50.4 | 71.12 | 71.62 | 0.7% | 72.17 |
| **Ketchup wfk 20T** | 54.5 | 72.94 | 72.75 | 0.3% | 73.01 |
| **Curry wfk 20U** | 44.7 | 62.54 | 62.98 | 0.7% | 63.35 |
| **Soy sauce wfk 20V** | 29.6 | 74.87 | 74.95 | 0.1% | 74.33 |
| **Chocolate wfk 20Z** | 48.2 | 69.61 | 69.91 | 0.4% | 69.07 |
| **Blood, milk, C EMPA 117** | 9.8 | 39.21 | 41.07 | 4.7% | 37.83 |

Anti-redeposition performance is presented in Table 4. The wash trial included a dosage of 0.2% ethoxy according to the surfactant (LDB1, LDBB, and so forth) and 0.05% sodium triphosphate STP builder. The percent difference (% Diff.) as an absolute value compares LDBB and LDB1.

**Table 4. Detergency Trial (Anti-redeposition Effect)**

| | **Reflectance (%) at 460 nm** | | | |
|---|---|---|---|---|
| **Test** | **Ethoxylate type** | | | |
| | **LDBB** | **LDB1** | **\|%Diff.\|** | **NP9** |
| **Anti-Redeposition** | **68.3** | **70.06** | **2.58** | **65.33** |

Properties of LDB1 and LDBB are similar and they are substantially equal in performance. Furthermore, detergency (stain removal and anti-redeposition) performance is slightly superior to that of NP9. Therefore, it can be concluded that two or more fatty alcohols can be blended and ethoxylated together. Ethoxylating each fatty alcohol individually and then blending requires additional facility and consumes more resources and time. The ethoxylated blend of alcohols generated according to the present disclosure effected a detergency comparable to a physical mixture of ethoxylated alcohols in that detergency performance for the same stains and same fabrics subjected to the same laundering was within 5%.

As further evidenced herein, the disclosed ratio of alcohols in the mixture altered certain physical and chemical properties of the surfactant composition. Inventive formulation LDB1 was carried out at a 1:1 ratio of the alcohols for ethoxylation. To illustrate the effect of the alcohol ratios, DA5 L7 surfactant samples were also prepared at 7:3 and 3:7 at 0.1 % concentration and evaluated for cloud point and wettability. Table 5 summarizes these results.

**Table 5. Physical and Chemical Properties for DA5 L7 at varying ratios.**

| **Test** | **Conc. 0.1%** | | | |
|---|---|---|---|---|
| | **DA5 (C₁₀):L7 (C₁₂₋₁₄) 1:1 LDB1** | **DA5 (C₁₀):L7 (C₁₂₋₁₄) 7:3** | **DA5 (C₁₀):L7 (C₁₂₋₁₄) 3:7** | **NP9** |
| **Cloud Point** | 49 | 35.6 | 42 | 54 |
| **Wettability (secs)** | 9.14 | 6.04 | 11 | 9.24 |

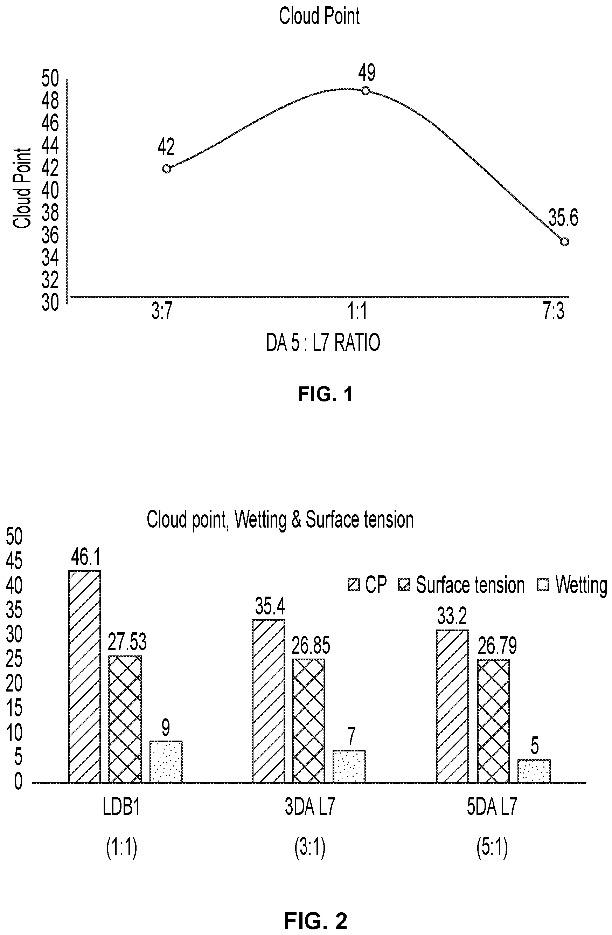

The cloud point observed for LDB1 is greater than that for both the 7:3 ratio and 3:7 ratio. The LDB1 cloud point value is also closest to that of conventional surfactant NP9. Wettability for LDB1 is also similarly comparable to that of NP9. FIG. 1 is a graphical representation of the cloud point plotted as a function of the ratios and indicated a critical cloud point value correlating to the 1:1 ratio.

As a further comparative illustration, isodecyl alcohol (IDA) and lauryl alcohol (C₁₂₋₁₄ alcohol) were combined in a single reaction vessel and the mixture was then ethoxylated in the pilot plant of 30L capacity to provide formulations with varying amounts of the ethoxylated alcohol mixtures, namely 5DA L7 (5:1) and 3DA L7 (3:1), prepared according to methods of the present disclosure. Ethoxylation conditions proceeded as in Table 1 above. The reaction sequence comprised the combination of alcohol precursors IDS and C₁₂₋₁₄ alcohol with a catalyst (potassium hydroxide as a 50% aqueous solution, in an amount of 0.2%), dehydration, addition of ethylene oxide, digestion, a process control check, and cooling and neutralization. Table 6 provides the cloud point, surface tension, and wettings values for 5DA L7 (5:1) and 3DA L7 (3:1) compared to the 1:1 sample (LDB1). FIG. 2 provides a graphical representation of these values.

**Table 6. Cloud point, surface tension, and wetting values.**

| **L7:DAS** | **Cloud Point (°C)** | **Surface tension dyn/cm** | **Wetting (secs)** |
|---|---|---|---|
| LDB1 (1:1) | 48.3 | 27.53 | 9 |
| 3:1 | 35.4 | 26.85 | 7 |
| 5:1 | 33.2 | 26.79 | 5 |

As shown, the cloud point of 1:1 ratio (L7:DA5, as LDB1) was greater than that of the formulations at ratios 3:1 and 5:1, while maintaining a comparable surface tension (within 3%) and wetting. Without wishing to be bound by any particular theory, the higher cloud point suggested that the disclosed laundry formulation exhibited better storage stability than the comparative samples at different ratios.

Detergency properties of LDB1, 3DA L7 (3:1), and 5DA L7 (5:1) were measured using a Tergotometer and Konica Minolta spectrophotometer. Values for reflectance at 460 nm are presented as a graphical representation in FIG. 2. Laundry wash trials were conducted using a tergotometer and deionized water at 40 °C and at 50 °C. The wash trials were performed at 200 revolutions per minute (rpm) for 30 minutes with a load of 25 grams per liter (g/L) for the varying samples. The wash trial included a dosage of 0.2% ethoxy and 0.05% sodium triphosphate STP builder. The laundered samples were rinsed and dried at room temperature. Reflectance was obtained at 460 nm for each sample of laundered fabric according to the stain types. The reflectance measurements were performed on a Konica Minolta Spectrophotometer 3610A. All measurements are made after calibrating the instrument against a black standard and white standard of known reflectance. Measurements were made according to the reference standard ASTM D 3050 - 07. Reflectance values of all the fabrics were obtained before and after washing. The reference stains were Pigment Lanolin wfk 20 C, Pigment Sebum wfk 20 D, Egg Yolk wfk 20 EG, and Lip stick wfk 20 LS. Results at 40 °C are presented in Table 7.

**Table 7. Detergency Trial at 40 °C**

| **Method : Tergotometer, DIW, 40 °C, 200 rpm, 30 min, load 25g/L, dosage 0.2%, RT drying** | | | | | | |
|---|---|---|---|---|---|---|
| **Stain type** | **Reflectance (%) at 460 nm** | | | | | |
| | **U/W** | **Ethoxylate type** | | | | |
| | | **LDB1 (Inventive)** | **3DA L7** | **5DA L7** | **\|% Diff.\| LDB1 and 3DA L7** | **\|% Diff.\| LDB1 and 5DA L7** |
| **Pigment Lanolin wfk 20C** | NA | 75.4 | 75.54 | 75.58 | 0.185332 | 0.23815824 |
| **Pigment Sebum wfk 20D** | NA | 86.79 | 87.32 | 87.18 | 0.606963 | 0.44735031 |
| **Egg Yolk wfk 20EG** | NA | 89.96 | 90.12 | 90.12 | 0.177541 | 0.17754106 |
| **Lip stick wfk 20LS** | NA | 85.28 | 84.48 | 84.9 | 0.94697 | 0.44758539 |

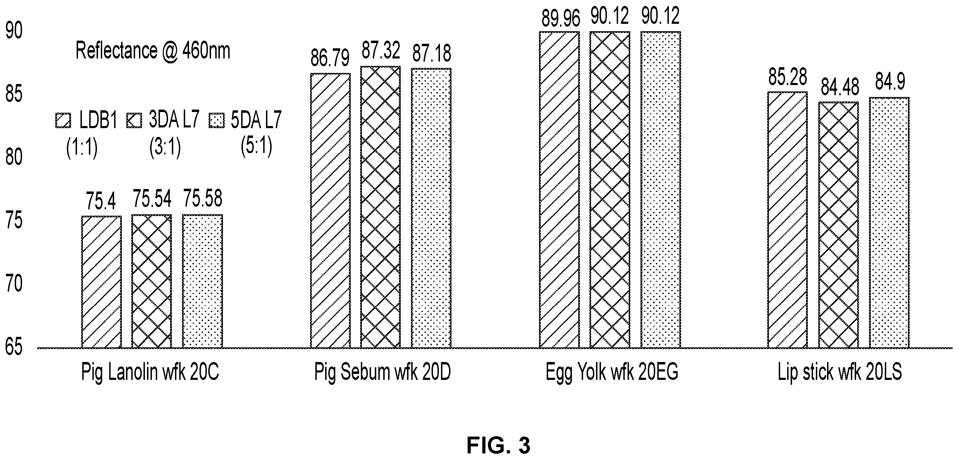

FIG. 3 provides a graphical representation of the reflectance observed at 460 nm for LDB1, 3DA L7 (3:1), and 5DA L7 (5:1) at 40 °C.

As shown, the 1:1 LDB1 sample provided reflectance results comparable to those achieved using 3DA L7 (3:1) and 5DA L7 (5:1). The results were comparable in that the LDB1 values were within 3%, and more specifically, within 1% of the reflectance values for 3DA L7 (3:1) and 5DA L7 (5:1). Results observed at 50 °C however demonstrate the improvement observed with inventive formulation LDB1 (1:1). Table 8 presents the reflectance values obtained for the 50 °C wash trial.

**Table 8. Detergency Trial at 50 °C**

| **Method : Tergotometer, DIW, 50 °C, 200 rpm, 30 min, load 25g/L, dosage 0.2%, RT drying** | | | | |
|---|---|---|---|---|
| **Stain type** | **Reflectance (%) at 460 nm** | | | |
| | **U/W** | **Ethoxylate type** | | |
| | | **LDB1 (Inventive)** | **3DA L7** | **5DA L7** |
| **Pigment Lanolin wfk 20C** | NA | 81.93 | 81.69 | 79.95 |
| **Pigment Sebum wfk 20D** | NA | 85.69 | 85.45 | 85.5 |
| **Egg Yolk wfk 20EG** | NA | 88.39 | 87.47 | 88.02 |
| **Lip stick wfk 20LS** | NA | 86.87 | 86.6 | 85.69 |

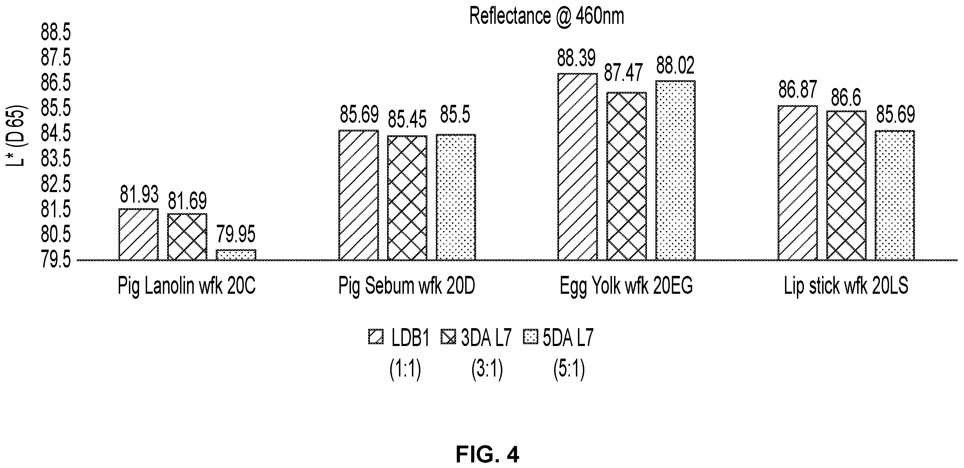

Surprisingly, for each stain type treated, the LDB1 formulation exhibited the highest observed reflectance for trials performed at 50 °C as demonstrated in FIG. 4.

As depicted, the 1:1 ratio of surfactant (DA5 L7, LDB1) exhibited higher reflectance and thus a better detergency performance at the higher temperature compared to the other ratios (3:1 and 5:1) for the down selected standard stains. Without wishing to be bound by any particular theory, the higher cloud point of LDB1 may have led to or may have contributed to detergency enhancement.

The patentable scope of the disclosure is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method of forming a surfactant composition, the method comprising:
a. combining
i. a C₁₂₋₁₄ aliphatic straight chain alcohol, and
ii. a C₁₀ aliphatic branched chain alcohol
in a single reaction vessel to provide an alcohol mixture wherein the C₁₂₋₁₄ aliphatic straight chain alcohol and the C10 aliphatic branched chain alcohol are combined in a weight ratio of from about 0.8:1 to 1.4:1; and
b. ethoxylating the alcohol mixture to provide a surfactant composition,
wherein:
a stained fabric subjected to a laundry wash trial with the surfactant composition exhibits a reflectance at 460 nm that is within 5% of a reflectance observed at 460 nm on a stained fabric subjected to a laundry wash trial with a reference composition,
the reference composition comprises a physical mixture of ethoxylated C₁₀ aliphatic branched chain alcohol having 5 moles ethoxylate and ethoxylated C₁₂₋₁₄ aliphatic straight chain alcohol having 7 moles ethoxylate in a weight ratio of 1:1,
the laundry wash trial comprises laundering of the fabric in deionized water at 40 °C with a dosage of 0.2 % of the surfactant or reference composition in accordance with ASTM-3050-70, and
the stained fabric is a cotton polyester blend.

2. The method of claim 1, wherein the ethoxylating proceeds until a hydroxyl value of 122-132 in the ethoxylated alcohol mixture is obtained when determined in accordance with ASTM E1899-08.

3. The method of claim 1, wherein the C₁₀ aliphatic branched chain alcohol and C₁₂₋₁₄ aliphatic straight chain alcohol are combined in a 1:1 weight ratio.

4. The method of any one of claims 1-3, wherein the ethoxylating comprises combining the alcohol mixture with a catalyst and reacting the resulting mixture with about 2 moles of ethylene oxide to about 12 moles of ethylene oxide per 1 mole of alcohol.

5. The method of any one of claims 1-4, wherein the ethoxylating comprises combining the alcohol mixture with a catalyst and reacting the resulting mixture with about 6 moles of ethylene oxide per 1 mole of alcohol.

6. The method of any one of claims 1-5, wherein the ethoxylating proceeds at a temperature of about 150 °C to about 170 °C.

7. The method of any one of claims 1-6, wherein the ethoxylating proceeds at a pressure of about 2 bar to about 3.5 bar.

8. The method of any one of claims 1-7, wherein the surfactant composition exhibits a cloud point in a 1% aqueous solution that is within 5% of a cloud point observed for a reference composition in a 1% aqueous solution, wherein the reference composition comprises a physical mixture of ethoxylated C₁₀ aliphatic branched chain and ethoxylated C₁₂₋₁₄ aliphatic straight chain alcohols in a 1:1 weight ratio, and when measured in accordance with ASTM D 2024-09.

9. The method of any one of claims 1-8, wherein the surfactant composition has a pH value that is within 3% of a pH observed for a reference composition, wherein the reference composition comprises a physical mixture of ethoxylated C₁₀ aliphatic branched chain and ethoxylated C₁₂ aliphatic straight chain alcohols in a 1:1 weight ratio, when measured in accordance with ASTM D1293-12.

10. The method of any one of claims 1-8, wherein the surfactant composition exhibits a surface tension that is within 3% of a surface tension measured for a reference composition, wherein the reference composition comprises a physical mixture of ethoxylated C₁₀ aliphatic branched chain and ethoxylated C₁₂ aliphatic straight chain alcohols in a 1:1 weight ratio, when measured in accordance with ASTM D1331-4.

11. The method of any one of claims 1-8, wherein the stained fabric subjected to a laundry wash trial with the surfactant composition exhibits a reflectance at 460 nm that is within 3% of a reflectance observed at 460 nm on a stained fabric subjected to the laundry wash trial with the reference composition.

12. The method of any one of claims 1-8, wherein a stained fabric subjected to a laundry wash trial with the surfactant composition exhibits a reflectance at 460 nm that is greater than a reflectance observed at 460 nm on a stained fabric subjected to a laundry wash trial with a reference composition, wherein the reference composition comprises an ethoxylated mixture of C₁₀ aliphatic branched chain alcohol having 5 moles ethoxylate and ethoxylated C₁₂₋₁₄ aliphatic straight chain alcohol having 7 moles ethoxylates, or wherein the reference composition comprises an ethoxylated mixture of C₁₀ aliphatic branched chain alcohol having 3 moles ethoxylate and C₁₂₋₁₄ aliphatic straight chain alcohol having 7 moles ethoxylates, wherein the laundry wash trial comprises laundering of the fabric in deionized water at 50 °C with a dosage of 0.4 % of the surfactant or reference composition, and wherein the stained fabric comprises pigment lanolin wfk 20C, pigment sebum wfk 20D, egg yolk 20EG, or lipstick wfk 20 LS.

13. The method of any of claims 1-12, wherein the method is free of distillation.

14. The method of any of claims 1-13, wherein the stained fabric comprises a stain of any one of the following stain types: soy sauce pigment; olive oil WFK 20B; pigment lanolin WFK 20C; pigment sebum WFK 20D; egg yolk WFK 20EG; used motor oil WFK 20GM; coffee WFK 20K; red grape WFK 20LIU; lip stick WFK 20LS; makeup WFK 20MU; pigment egg WFK 20N; pigment vegetable fat WFK 20PF; soot mineral oil WFK 20RM; tomato beef sauce WFK 20SG; spinach WFK 20SP; ketchup WFK 20T; curry WFK 20U; soy sauce WFK 20V; chocolate WFK 20Z; blood-milk-C EMPA 117; or a combination thereof, for soiled test fabrics according to WFK Testgewebe GmbH or blood-milk C EMPA 117 according to EMPA of Switzerland standard stain type.

## Patentansprüche

1. Verfahren zum Bilden einer Tensidzusammensetzung, wobei das Verfahren Folgendes umfasst:
a. Zusammenbringen von
i. einem geradkettigen aliphatischen C₁₂₋₁₄-Alkohol und
ii. einem verzweigten aliphatischen C₁₀-Alkohol in einem einzigen Reaktionsgefäß zur Bereitstellung einer Alkoholmischung, in der der geradkettige aliphatische C₁₂₋₁₄-Alkohol und der verzweigte aliphatische C₁₀-Alkohol in einem Gewichtsverhältnis von etwa 0,8:1 bis 1,4:1 zusammengebracht werden; und
b. Ethoxylieren der Alkoholmischung zur Bereitstellung einer Tensidzusammensetzung,
wobei:
ein beschmutzter Stoff, der mit der Tensidzusammensetzung in einem Versuchswaschgang für Wäsche gewaschen wird, eine Reflexion bei 460 nm aufweist, die innerhalb von 5% von einer Reflexion liegt, die bei 460 nm an einem beschmutzten Stoff beobachtet wird, der mit einer Bezugszusammensetzung in einem Versuchswaschgang für Wäsche gewaschen wird,
die Bezugszusammensetzung eine physikalische Mischung aus ethoxyliertem verzweigtem aliphatischem C₁₀-Alkohol mit 5 Mol Ethoxylat und
ethoxyliertem geradkettigem aliphatischem C₁₂₋₁₄-Alkohol mit 7 Mol Ethoxylat in einem Gewichtsverhältnis von 1:1 umfasst,
der Versuchswaschgang für Wäsche ein Waschen des Stoffs in demineralisiertem Wasser bei 40 °C mit einer Dosierung der Tensid- oder Bezugszusammensetzung von 0,2 % gemäß ASTM-3050-70 umfasst und
der beschmutzte Stoff eine Baumwolle-Polyester-Mischung ist.

2. Verfahren nach Anspruch 1, wobei die Ethoxylierung erfolgt, bis in der ethoxylierten Alkoholmischung bei Ermittlung gemäß ASTM E1899-08 ein Hydroxylwert von 122 - 132 erhalten wird.

3. Verfahren nach Anspruch 1, wobei der verzweigte aliphatische C₁₀-Alkohol und der geradkettige aliphatische C₁₂₋₁₄-Alkohol in einem Gewichtsverhältnis von 1:1 zusammengebracht werden.

4. Verfahren nach einem der Ansprüche 1-3, wobei die Ethoxylierung ein Zusammenbringen der Alkoholmischung mit einem Katalysator und Reagierenlassen der entstehenden Mischung mit ungefähr 2 Mol Ethylenoxid bis ungefähr 12 Mol Ethylenoxid pro 1 Mol Alkohol umfasst.

5. Verfahren nach einem der Ansprüche 1-4, wobei die Ethoxylierung ein Zusammenbringen der Alkoholmischung mit einem Katalysator und Reagierenlassen der entstehenden Mischung mit ungefähr 6 Mol Ethylenoxid pro 1 Mol Alkohol umfasst.

6. Verfahren nach einem der Ansprüche 1-5, wobei die Ethoxylierung bei einer Temperatur von ungefähr 150 °C bis ungefähr 170 °C erfolgt.

7. Verfahren nach einem der Ansprüche 1-6, wobei die Ethoxylierung bei einem Druck von ungefähr 2 Bar bis ungefähr 3,5 Bar erfolgt.

8. Verfahren nach einem der Ansprüche 1-7, wobei die Tensidzusammensetzung einen Trübungspunkt in einer 1%-igen wässrigen Lösung aufweist, der innerhalb von 5% eines Trübungspunkts liegt, der bei einer Bezugszusammensetzung in einer 1%-igen wässrigen Lösung beobachtet wird, wobei die Bezugszusammensetzung eine physikalische Mischung aus ethoxyliertem verzweigtem aliphatischem C₁₀-Alkohol und ethoxyliertem geradkettigem aliphatischem C₁₂₋₁₄-Alkohol in einem Gewichtsverhältnis von 1:1 umfasst, und bei Messung gemäß ASTM D 2024-09.

9. Verfahren nach einem der Ansprüche 1-8, wobei die Tensidzusammensetzung einen pH-Wert aufweist, der innerhalb von 3% eines pH-Werts liegt, der bei einer Bezugszusammensetzung beobachtet wird, wobei die Bezugszusammensetzung eine physikalische Mischung aus ethoxyliertem verzweigtem aliphatischem C₁₀-Alkohol und ethoxyliertem geradkettigem aliphatischem C₁₂-Alkohol in einem Gewichtsverhältnis von 1:1 bei Messung gemäß ASTM D1293-12 umfasst.

10. Verfahren nach einem der Ansprüche 1-8, wobei die Tensidzusammensetzung eine Oberflächenspannung aufweist, die innerhalb von 3% einer Oberflächenspannung liegt, die bei einer Bezugszusammensetzung gemessen wird, wobei die Bezugszusammensetzung eine physikalische Mischung aus ethoxyliertem verzweigtem aliphatischem C₁₀-Alkohol und ethoxyliertem geradkettigem aliphatischem C₁₂-Alkohol in einem Gewichtsverhältnis von 1:1 bei Messung gemäß ASTM D 1331-4 umfasst.

11. Verfahren nach einem der Ansprüche 1-8, wobei der beschmutzte Stoff, der mit der Tensidzusammensetzung in einem Versuchswaschgang für Wäsche gewaschen wird, eine Reflexion bei 460 nm aufweist, die innerhalb von 3% von einer Reflexion liegt, die bei 460 nm an einem beschmutzten Stoff beobachtet wird, der mit der Bezugszusammensetzung in dem Versuchswaschgang für Wäsche gewaschen wird.

12. Verfahren nach einem der Ansprüche 1-8, wobei ein beschmutzter Stoff, der mit der Tensidzusammensetzung in einem Versuchswaschgang für Wäsche gewaschen wird, eine Reflexion bei 460 nm aufweist, die höher ist als eine Reflexion, die bei 460 nm an einem beschmutzten Stoff beobachtet wird, der mit einer Bezugszusammensetzung in einem Versuchswaschgang für Wäsche gewaschen wird, wobei die Bezugszusammensetzung eine ethoxylierte Mischung aus verzweigtem aliphatischem C₁₀-Alkohol mit 5 Mol Ethoxylat und ethoxyliertem geradkettigem aliphatischem C₁₂₋₁₄-Alkohol mit 7 Mol Ethoxylaten umfasst, oder wobei die Bezugszusammensetzung eine ethoxylierte Mischung aus verzweigtem aliphatischem C₁₀-Alkohol mit 3 Mol Ethoxylat und ethoxyliertem geradkettigem aliphatischem C₁₂₋₁₄-Alkohol mit 7 Mol Ethoxylaten umfasst, wobei der Versuchswaschgang für Wäsche ein Waschen des Stoffs in demineralisiertem Wasser bei 50 °C mit einer Dosierung der Tensid- oder Bezugszusammensetzung von 0,4 % umfasst, und wobei der beschmutzte Stoff Pigment/Lanolin WFK 20C, Pigment/Talg WFK 20D, Eigelb 20EG oder Lippenstift WFK 20 LS umfasst.

13. Verfahren nach einem der Ansprüche 1-12, wobei das Verfahren keine Destillation umfasst.

14. Verfahren nach einem der Ansprüche 1-13, wobei der beschmutzte Stoff eine Anschmutzung von einer beliebigen der folgenden Anschmutzungsarten umfasst: Sojasauce/Pigment; Olivenöl WFK 20B; Pigment/Lanolin WFK 20C; Pigment/Talg WFK 20D; Eigelb WFK 20EG; Motoraltöl WFK 20GM; Kaffee WFK 20K; rote Weintraube WFK 20LIU; Lippenstift WFK 20LS; Makeup WFK 20MU; Pigment/Ei WFK 20N; Pigment/tierisches Fett WFK 20PF; Ruß/Mineralöl WFK 20RM; Sauce aus Tomate und Rindfleisch WFK 20SG; Spinat WFK 20SP; Ketchup WFK 20T; Curry WFK 20U; Sojasauce WFK 20V; Schokolade WFK 20Z; Blut-Milch-Tusche EMPA 117 oder eine Kombination daraus, für beschmutztes Testgewebe gemäß WFK Testgewebe GmbH oder Blut-Milch-Tusche EMPA 117 gemäß Standard-Anschmutzung EMPA, Schweiz.

## Revendications

1. Procédé de formation d'une composition de tensioactif, le procédé comprenant :
a. la combinaison
i. d'un alcool aliphatique à chaîne droite en C₁₂ à C₁₄, et
ii. d'un alcool aliphatique à chaîne ramifiée en C₁₀ dans un seul récipient réactionnel pour former un mélange d'alcools dans lequel l'alcool aliphatique à chaîne droite en C₁₂ à C₁₄ et l'alcool aliphatique à chaîne ramifiée en C₁₀ sont combinés en un rapport en poids d'environ 0,8/1 à 1,4/1 ; et
b. l'éthoxylation du mélange d'alcools pour former une composition de tensioactif,
dans lequel :
une étoffe salie soumise à un essai de lavage du linge avec la composition de tensioactif présente un coefficient de réflexion à 460 nm qui est dans les 5 % du coefficient de réflexion observé à 460 nm sur une étoffe tachée soumise à un essai de lavage du linge avec une composition de référence,
la composition de référence comprend un mélange physique d'alcool aliphatique à chaîne ramifiée en C₁₀ éthoxylé ayant 5 moles d'éthoxylate et d'alcool aliphatique à chaîne droite en C₁₂ à C₁₄ éthoxylé ayant 7 moles d'éthoxylate, en un rapport en poids de 1/1,
l'essai de lavage du linge comprend le lavage de l'étoffe dans de l'eau désionisée à 40°C avec une dose de 0,2 % du tensioactif ou de la composition de référence conformément à la norme ASTM-3050-70, et
l'étoffe salie est en un mélange de coton et de polyester.

2. Procédé selon la revendication 1, dans lequel l'éthoxylation se déroule jusqu'à ce qu'un indice d'hydroxyle de 122 à 132 dans le mélange d'alcools éthoxylés soit obtenu, quand il est déterminé conformément à la norme ASTM E1899-08.

3. Procédé selon la revendication 1, dans lequel l'alcool aliphatique à chaîne ramifiée en C₁₀ et l'alcool aliphatique à chaîne droite en C₁₂ à C₁₄ sont combinés en un rapport en poids de 1/1.

4. Procédé selon l'une quelconque des revendications 1 à 3, dans lequel l'éthoxylation comprend la combinaison du mélange d'alcools avec un catalyseur et la réaction du mélange résultant avec environ 2 moles d'oxyde d'éthylène à environ 12 moles d'oxyde d'éthylène par mole d'alcool.

5. Procédé selon l'une quelconque des revendications 1 à 4, dans lequel l'éthoxylation comprend la combinaison du mélange d'alcools avec un catalyseur et la réaction du mélange résultant avec environ 6 moles d'oxyde d'éthylène par mole d'alcool.

6. Procédé selon l'une quelconque des revendications 1 à 5, dans lequel l'éthoxylation se déroule à une température d'environ 150°C à environ 170°C.

7. Procédé selon l'une quelconque des revendications 1 à 6, dans lequel l'éthoxylation se déroule sous une pression d'environ 2 bar à environ 3,5 bar.

8. Procédé selon l'une quelconque des revendications 1 à 7, dans lequel la composition de tensioactif présente un point de trouble dans une solution aqueuse à 1 % qui est dans les 5 % du point de trouble observé pour une composition de référence dans une solution aqueuse à 1 %, dans lequel la composition de référence comprend un mélange physique d'alcool aliphatique à chaîne ramifiée en C₁₀ éthoxylé et d'alcool aliphatique à chaîne droite en C₁₂ à C₁₄ éthoxylé en un rapport en poids de 1/1, quand il est mesuré conformément à la norme ASTM D 2024-09.

9. Procédé selon l'une quelconque des revendications 1 à 8, dans lequel la composition de tensioactif a une valeur de pH qui est dans les 3 % du pH observé pour une composition de référence, dans lequel la composition de référence comprend un mélange physique d'alcool aliphatique à chaîne ramifiée en C₁₀ éthoxylé et d'alcool aliphatique à chaîne droite en C₁₂ éthoxylé en un rapport en poids de 1/1, quand elle est mesurée conformément à la norme ASTM D1293-12.

10. Procédé selon l'une quelconque des revendications 1 à 8, dans lequel la composition de tensioactif présente une tension de surface qui est dans les 3 % de la tension de surface mesurée pour une composition de référence, dans lequel la composition de référence comprend un mélange physique d'alcool aliphatique à chaîne ramifiée en C₁₀ éthoxylé et d'alcool aliphatique à chaîne droite en C₁₂ éthoxylé en un rapport en poids de 1/1, quand elle est mesurée conformément à la norme ASTM D1331-4.

11. Procédé selon l'une quelconque des revendications 1 à 8, dans lequel l'étoffe salie soumise à un essai de lavage du linge avec la composition de tensioactif présente un coefficient de réflexion à 460 nm qui est dans les 3 % du coefficient de réflexion observé à 460 nm sur une étoffe tachée soumise à un essai de lavage du linge avec la composition de référence.

12. Procédé selon l'une quelconque des revendications 1 à 8, dans lequel l'étoffe salie soumise à un essai de lavage du linge avec la composition de tensioactif présente un coefficient de réflexion à 460 nm qui est supérieur au coefficient de réflexion observé à 460 nm sur une étoffe salie soumise à un essai de lavage du linge avec une composition de référence, dans lequel la composition de référence comprend un mélange éthoxylé d'alcool aliphatique à chaîne ramifiée en C₁₀ ayant 5 moles d'éthoxylate et d'alcool aliphatique à chaîne droite en C₁₂ à C₁₄ éthoxylé ayant 7 moles d'éthoxylate, ou dans lequel la composition de référence comprend un mélange éthoxylé d'alcool aliphatique à chaîne ramifiée en C₁₀ ayant 3 moles d'éthoxylate et d'alcool aliphatique à chaîne droite en C₁₂ à C₁₄ ayant 7 moles d'éthoxylate, dans lequel l'essai de lavage du linge comprend le lavage de l'étoffe dans de l'eau désionisée à 50°C avec une dose de 0,4 % du tensioactif ou de la composition de référence, et dans lequel l'étoffe salie comprend du pigment lanoline wfk 20C, du pigment sébum wfk 20D, du jaune d'œuf 20EG, ou du rouge à lèvres wfk 20 LS.

13. Procédé selon l'une quelconque des revendications 1 à 12, lequel procédé est exempt de distillation.

14. Procédé selon l'une quelconque des revendications 1 à 13, dans lequel l'étoffe salie comprend une tache parmi n'importe lesquelles des types de taches suivants : pigment de sauce soja ; huile d'olive WFK 20B ; pigment lanoline WFK 20C ; pigment sébum WFK 20D ; jaune d'œuf WFK 20EG ; huile moteur usagée WFK 20GM ; café WFK 20K ; raisin rouge WFK 20LIU ; rouge à lèvres WFK 20LS ; maquillage WFK 20MU ; pigment œuf WFK 20N ; pigment graisse végétale WFK 20PF ; huile minérale avec suie WFK 20RM ; sauce tomate au bœuf WFK 20SG ; épinard WFK 20SP ; ketchup WFK 20T ; curry WFK 2U ; sauce soja WFK 20V ; chocolat WFK 20Z ; sang-lait-C EMPA 117 ; ou une de leurs combinaisons, pour étoffes de test salies conformément à WFK Testgewebe GmbH ou le type de tache normalisé sang-lait C EMPA 117 conformément à l'EMPA en Suisse.
